# EUROPEAN PATENT SPECIFICATION

(11) **EP 3 458 145 B1**
(45) Date of publication and mention of the grant of the patent: **25.03.2020**
(21) Application number: 17735626.8
(22) Date of filing: 18.05.2017
(51) Int. Cl.: A61M 39/10, A61M 39/26

(54) **VALVED CONNECTOR FOR MEDICAL LINES**
VERBINDER MIT VENTIL FÜR MEDIZINISCHE LEITUNGEN
RACCORD À SOUPAPE POUR LIGNES MÉDICALES

(30) Priority: 19.05.2016 IT UA20163611
(43) Date of publication of application: 27.03.2019
(73) Proprietor: Industrie Borla S.p.A., 10024 Moncalieri (Torino) (IT)
(72) Inventor: GUALA, Gianni, 10133 Torino (IT)
(74) Representative: Buzzi, Franco
(86) International application number: PCT/IB2017/052943
(87) International publication number: WO 2017/199203

(56) References cited:
- EP-A1- 1 747 796
- WO-A1-03/030986
- WO-A1-2009/095760
- US-A- 5 269 771

## Description

### Field of the invention

The present invention regards connectors for medical lines, particularly for hemodialysis lines, of the type comprising a male luer lock valved fitting and a female luer fitting that can be engaged with the male luer lock fitting.

### State of the prior art

Document WO 2009/095760 (EP-2237830B1) on behalf of the Applicant reveals a connector thus made in which the male luer lock valved fitting comprises an outer body, an inner tubular element axially displaceable with respect to the outer body between a receded position and an advanced position, and a hollow element made of elastic material interposed between the outer body and the inner tubular element and having a transversal end wall formed with a pre-cut: in the receded position of the inner tubular element, the pre-cut is closed, so as to cut off the flow between the female luer lock fitting and the male luer lock fitting. The connector further comprises a rotatable ring nut on the outer body of the male luer lock fitting and having an inner threading that can be screwed with an outer thread of the female luer lock fitting, after the latter is axially engaged on the inner tubular element.

When the ring nut is screwed, the inner tubular element is displaced from the receded position towards the advanced position compressing the hollow element made of elastic material and deforming the transversal end wall of the latter so as to open the pre-cut.

Furthermore, in this known valved connector, the inner luer cone of the female luer lock fitting is suitable to be engaged by friction with the outer luer cone of the inner tubular element before the screwing of the rotatable ring nut, and to maintain the friction engagement after unscrewing the rotatable ring nut.

When the slidable inner tubular element is in the receded closing condition, there occurs a given axial distance between the end thereof and the transversal wall of the elastic hollow element. According to this arrangement, the opening of the pre-cut and thus the flow path between the female luer lock fitting and the male luer lock valved fitting, occurs gradually, thus - in some applications and in hemodialysis lines in particular - it can give rise to serious inconveniences for the patient. The closure of the pre-cut also occurs gradually following the unscrewing of the rotatable ring nut.

### Summary of the invention

The object of the present invention is to provide a valved connector of the type described above in which the opening of the flow between the female luer lock fitting and the male luer lock valved fitting, as well as the relative reclosing, are made in an ON/OFF switch-like manner.

According to the invention, this object is obtained due to the fact that, in a valved connector as defined in the pre-characterised part of claim 1, in the aforementioned receded position said inner tubular element of the male luer lock fitting is in substantial contact with said transversal end wall of the hollow element made of elastic material thus, when the inner luer cone of the female luer lock fitting is engaged by friction with said inner tubular element axially pushing it even before the unscrewing of said rotatable ring nut with the female luer lock fitting, said pre-cut is instantly fully opened, and when said ring nut ceases the unscrewing from the female luer lock fitting, said pre-cut fully re-closes instantly.

### Brief description of the drawings

The invention will now be described in detail with reference to the attached drawings, provided purely by way of non-limiting example, wherein:
- figure 1 is a longitudinal sectional view of a valved connector according to the invention, represented in the disabled condition i.e. the closure of the flow,
- figure 2 is a view analogous to figure 1 showing the valved connector in the previously enabled condition i.e. the opening of the flow, with the threaded coupling not engaged, and
- figure 3 is a view analogous to figures 1 and 2 showing the valved connector in a condition, still enabled, subsequent to the one of figure 2 and with the threaded coupling engaged.

### Detailed description of the invention

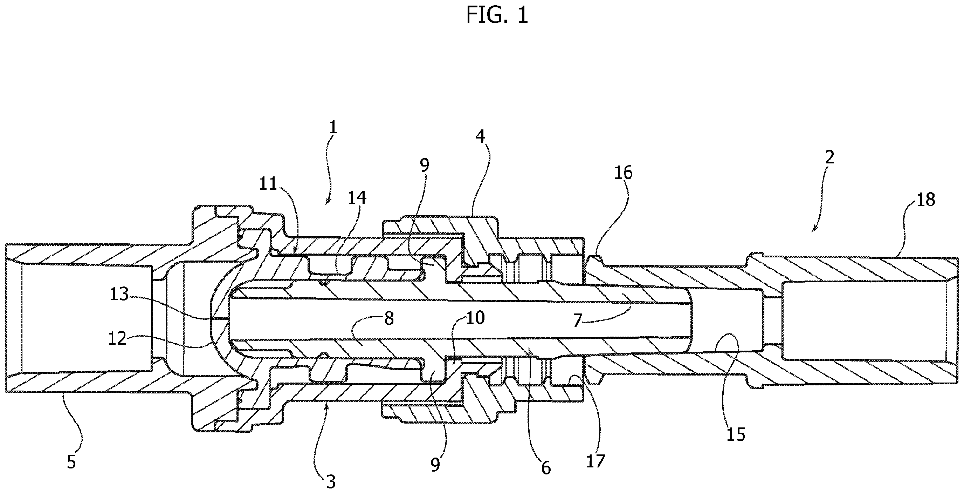

Initially with reference to figure 1, the connector for medical lines according to the invention comprises a male luer lock valved fitting 1 and a female luer lock fitting 2 that can be axially engaged with the male luer lock fitting 1.

The male luer lock fitting 1 comprises an outer tubular body 3 having a proximal end on which a ring nut 4 is coaxially rotatable and a distal end which forms a connector 5 intended to be connected to a section of the medical line, for example a duct connected to a hemodialysis equipment.

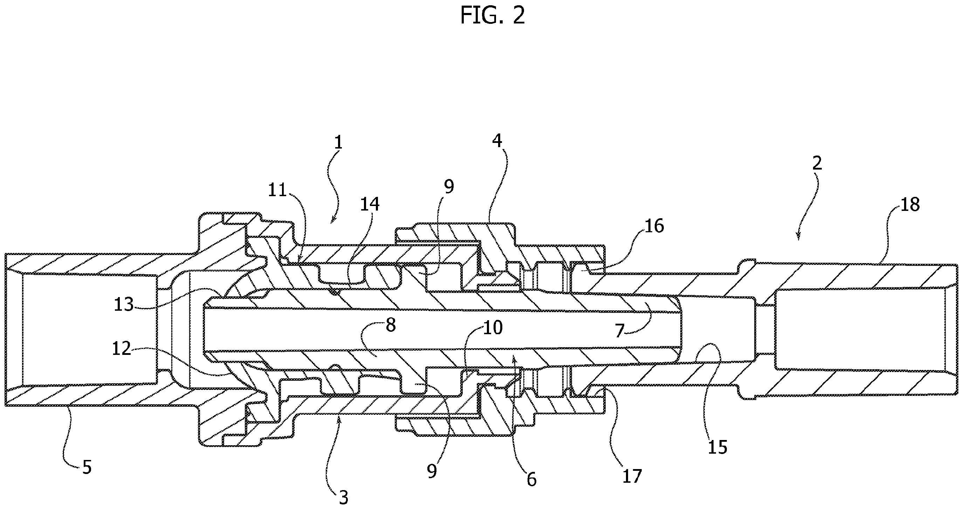

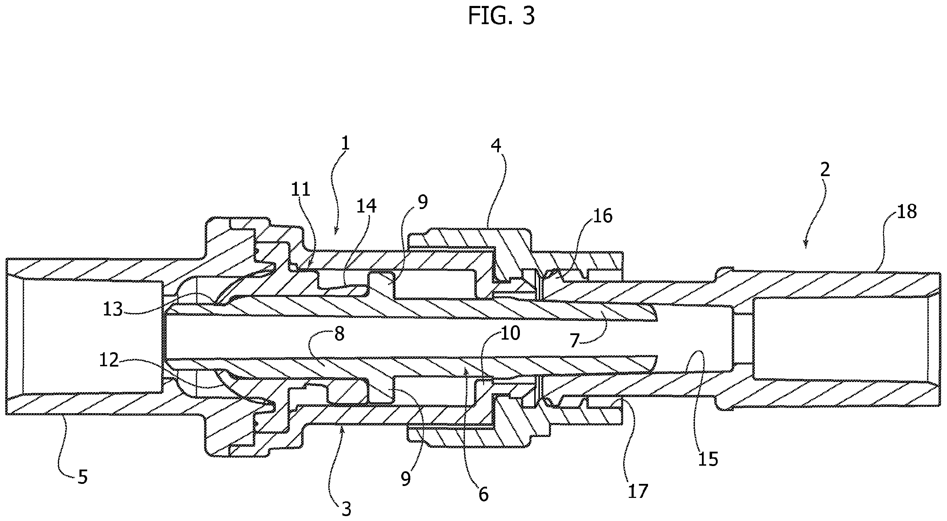

The male luer lock fitting 1 further comprises an inner tubular element 6 axially mounted slidable, with respect to the outer tubular body 3, between a receded position represented in figure 1 and a fully advanced position in the direction of the connector 5 represented in figure 3, passing through the partly advanced position illustrated in figure 2.

The inner tubular element 6 has a portion 7 projecting outside the rotatable ring nut 4 and shaped like an outer luer cone, and - on the opposite side - a portion 8 having outer radial projections 9 slidably but not rotatably coupled with the outer body 3. The projections 9, in the receded position of the inner tubular element 6, abut against an inner annular stop flange 10 of the outer body 3, and such receded position of the inner tubular element 6 is maintained due to the action of a hollow element made of elastic material 11 which is interposed coaxially and substantially sealingly between the outer body 3 and the portion 8 of the inner tubular element 6. This element made of elastic material 11 substantially integrates three functions: a first function, as mentioned, for elastically pushing the inner tubular element 6 towards the receded position; a second function for the slidable sealing of the portion 8 of the tubular element 6; and a third function consisting in defining a valved flow path through the male luer lock fitting 1. For this purpose, the hollow element made of elastic material 11 has a transversal end wall 12 facing the connector 5 of the outer body 3 and formed with a central pre-cut 13. On the opposite end, the hollow element made of elastic material 11 rests against the radial appendages 9 of the inner tubular element 6, and - in the intermediate part thereof - it has a more elastically yieldable wall, formed with alternated helical ribs and grooves 14, for example according to the description provided for in document EP-1747796A1.

The pre-cut 13 is normally maintained hermetically closed due to a suitable radial preload of the transversal wall 12.

According to the distinctive characteristic of the invention, when the inner tubular element 6 is arranged with the radial appendages 9 resting against the inner annular stop flange 10, the free end of the portion 8 thereof is in immediate axial proximity and conveniently in substantial front contact with the transversal wall 12 of the hollow element made of elastic material 11.

The female luer lock fitting 2 conventionally has - on the one side - an inner luer cone 15 complementary to the outer luer cone of the portion 7 of the inner tubular element 6, and - on the other side - a connector 18 that can be connected to the medical line, for example to a duct connected to a patient subjected to dialysis. The portion 15 is formed externally with an end thread 16 that can be engaged with an inner threading 17 of the rotatable ring nut 4.

Figure 1 represents the condition in which the inner luer cone 15 of the female luer lock fitting 2 is engaged on the outer luer cone of the portion 7 of the inner tubular element 6, before the thread 16 is engaged with the threading 17 of the rotatable ring nut 4. In this condition, the conical surfaces 15 and 7 are friction-coupled to each other, and the outer tubular element 6 is in the receded position with the pre-cut 13 maintained closed. As previously mentioned, according to the distinctive characteristic of the invention, in this condition the inner tubular element 6 is arranged, at the free end of the portion 8 thereof, in substantial contact with the transversal wall 12 of the hollow element made of elastic material 11.

Should - even before the threading 17 of the ring nut 4 can engage the thread 16 of the female luer lock fitting 2 - the latter be axially pushed to the position represented in figure 2, the inner tubular element 6 is in turn axially pushed by the female luer lock fitting 2 towards the advanced position and fully opens the pre-cut 13, as represented in figure 2: thus, the flow path through the valved connector promptly opens even before the rotatable ring nut 14 is screwed on the female luer lock fitting 2.

When the thread 16 engages the threading 17 and the ring nut 4 is rotated being screwed on such thread 16, as represented in figure 3, the valved connector is already fully open: the inner tubular element 6 is arranged in a fully advanced position, with the portion 8 thereof abundantly projecting beyond the pre-cut 13 and the hollow element made of elastic material 11 fully axially compressed.

When the rotatable ring nut 4 is unscrewed, the inner tubular element 6 is firstly displaced to the intermediate position of figure 2, due to the return of the hollow element made of elastic material 11 towards the undeformed condition as well as the drawing - by friction - by the female luer lock fitting 2, until it returns to the fully receded position of figure 1 in which the outer radial projections 9 abut against the inner annular flange 10. Upon reaching such position, in which the ring nut 4 is fully unscrewed, the pre-cut 13 fully recloses instantly, while the inner luer cone 15 of the female luer lock fitting 2 and the outer luer cone 7 of the inner tubular element 6 remain mutually engaged by friction and thus can be axially mutually separated intentionally.

Thus, basically the valved connector according to the invention is advantageously provided with an operation of the ON-OFF type, i.e. with the full opening of the flow already before the screwing of the rotatable ring nut 4, and an equally full and immediate reclosing the flow as soon as the rotatable ring nut 4 ceases unscrewing from the female luer lock fitting 2.

Obviously, the construction details and the embodiments may widely vary with respect to what has been described and illustrated, without departing from the scope of protection of the present invention as described in the claims that follow.

## Claims

1. Valved connector for medical lines, particularly hemodialysis lines, comprising a male luer lock valved fitting (1) and a female luer lock fitting (2) that can be engaged with the male luer lock fitting (1), wherein the male luer lock fitting (1) comprises an outer body (3), an inner tubular element (6) displaceable axially and with respect to the outer body (3) between a receded position and an advanced position, and a hollow element made of elastic material (11) interposed between the outer body (3) and the inner tubular element (6) and having a transversal end wall (12) formed with a pre-cut (13) which - in the receded position of the inner tubular element (6) - is closed to cut off the flow between the female luer lock fitting (2) and the male luer lock fitting (1); the outer body (3) of the male luer lock fitting (1) being provided with a rotatable ring nut (4) having an inner threading (17) that can be screwed with an outer thread (16) of the female luer lock fitting (2), and said inner tubular element (6) being displaceable from the receded position towards the advanced position compressing said hollow element made of elastic material (11) and deforming said transversal end wall (12) so as to open said pre-cut (13), and the female luer lock fitting (2) having an inner luer cone (15) suitable to be axially engaged by friction with said inner tubular element (6) already before the screwing and even after the unscrewing of said rotatable ring nut (4) with respect to said outer thread (16) of the female luer lock fitting (2), **characterised in that** in the aforementioned receded position said inner tubular element (6) of the male luer lock valved fitting (1) is in immediate axial proximity and in front contact with said transversal end wall (12) of the hollow element made of elastic material (11) whereby, when said inner luer cone (15) is engaged by friction with said inner tubular element (6) so as to axially push it towards the advanced position even before said inner threading (17) of the rotatable ring nut (4) can engage said outer thread (16) of the female luer lock fitting (2), said pre-cut (13) fully opens, and when said ring nut (4) is fully unscrewed from the female luer lock fitting, (2) said pre-cut (13) fully recloses instantly.

## Patentansprüche

1. Verbinder mit Ventil für medizinische Leitungen, insbesondere Hämodialyseleitungen, umfassend einen männlichen Luer-Lock-Anschluss (1) mit Ventil und einen weiblichen Luer-Lock-Anschluss (2), der mit dem männlichen Luer-Lock-Anschluss (1) in Eingriff gebracht werden kann, wobei der männliche Luer-Lock-Anschluss (1) einen Außenkörper (3), ein inneres röhrenförmiges Element (6), das axial und in Bezug auf den Außenkörper (3) zwischen einer zurückgezogenen Stellung und einer vorgeschobenen Stellung verschiebbar ist, und ein Hohlelement (11) umfasst, das aus elastischem Material hergestellt ist, das zwischen dem Außenkörper (3) und dem inneren röhrenförmigen Element (6) angeordnet ist und das eine quer verlaufende Stirnwand (12) aufweist, die mit einer Vorstanzung (13) gebildet ist, die - in der zurückgezogenen Stellung des röhrenförmigen Elements (6) - geschlossen ist, um den Durchfluss zwischen dem weiblichen Luer-Lock-Anschluss (2) und dem männlichen Luer-Lock-Anschluss (1) zu unterbrechen; wobei der Außenkörper (3) des männlichen Luer-Lock-Anschlusses (1) mit einer drehbaren Ringmutter (4) versehen ist, die ein Innengewinde (17) aufweist, das mit einem Außengewinde (16) des weiblichen Luer-Lock-Anschlusses (2) verschraubt werden kann, und das innere röhrenförmige Element (6) aus der zurückgezogenen Stellung in Richtung der vorgeschobenen Stellung verschiebbar ist, wobei es das Hohlelement (11) zusammendrückt, das aus elastischem Material hergestellt ist, und die quer verlaufende Stirnwand (12) verformt, sodass die Vorstanzung (13) geöffnet wird, und wobei der weibliche Luer-Lock-Anschluss (2) einen inneren Luer-Konus (15) aufweist, der geeignet ist, schon vor dem Verschrauben und noch nach dem Losschrauben der drehbaren Ringmutter (4) in Bezug auf das Außengewinde (16) des weiblichen Luer-Lock-Anschlusses (2) reibschlüssig axial mit dem inneren röhrenförmigen Element (6) in Eingriff zu stehen, **dadurch gekennzeichnet, dass** in der vorgenannten zurückgezogenen Stellung das innere röhrenförmige Element (6) des männlichen Luer-Lock-Anschlusses (1) mit Ventil sich in unmittelbarer axialer Nähe zu und in Vorderseitenkontakt mit der quer verlaufenden Stirnwand (12) des Hohlelements (11) befindet, das aus elastischem Material hergestellt ist, wodurch sich, wenn der innere Luer-Konus (15) reibschlüssig mit dem inneren röhrenförmigen Element (6) in Eingriff gebracht wird, sodass er es axial in Richtung der vorgeschobenen Stellung schiebt, noch bevor das Innengewinde (17) der drehbaren Ringmutter (4) mit dem Außengewinde (16) des weiblichen Luer-Lock-Anschlusses (2) in Eingriff gelangen kann, die Vorstanzung (13) vollständig öffnet, und sich die Vorstanzung (13) unmittelbar wieder vollständig schließt, wenn die Ringmutter (4) vollständig von dem weiblichen Luer-Lock-Anschluss (2) losgeschraubt ist.

## Revendications

1. Connecteur à valve pour lignes médicales, en particulier des lignes d'hémodialyse, comprenant un raccord Lueur mâle à valve (1) et un raccord Lueur femelle (2) qui peut être mis en prise avec le raccord Lueur mâle (1), dans lequel le raccord Lueur mâle (1) comprend un corps externe (3), un élément tubulaire interne (6) axialement déplaçable et par rapport au corps externe (3) entre une position reculée et une position avancée, et un élément creux réalisé avec un matériau élastique (11) intercalé entre le corps externe (3) et l'élément tubulaire interne (6) et ayant une paroi d'extrémité transversale (12) formée avec une prédécoupe (13) qui - dans la position reculée de l'élément tubulaire interne (6) - est fermée pour arrêter l'écoulement entre le raccord Lueur femelle (2) et le raccord Lueur mâle (1) ; le corps externe (3) du raccord Lueur mâle (1) étant prévu avec un écrou annulaire rotatif (4) ayant un filetage interne (17) qui peut être vissé avec un filet externe (16) du raccord Lueur femelle (2), et ledit élément tubulaire interne (6) étant déplaçable de la position reculée vers la position avancée, comprimant ledit élément creux réalisé avec un matériau élastique (11) et déformant ladite paroi d'extrémité transversale (12) afin d'ouvrir ladite prédécoupe (13), et le raccord Lueur femelle (2) ayant un cône Lueur interne (15) approprié pour être mis en prise axialement par friction avec ledit élément tubulaire interne (6) déjà avant le vissage et même après le dévissage de l'écrou annulaire rotatif (4) par rapport audit filet externe (16) du raccord Lueur femelle (2), **caractérisé en ce que** dans la position reculée mentionnée précédemment, ledit élément tubulaire interne (6) du raccord Lueur mâle à valve (1) est à proximité axiale immédiate et en contact de face avec ladite paroi d'extrémité transversale (12) de l'élément creux réalisé à partir de matériau élastique (11) moyennant quoi, lorsque ledit cône Lueur interne (15) est mis en prise par friction avec ledit élément tubulaire interne (6) afin de le pousser vers la position avancée même avant que ledit filetage interne (17) de l'écrou annulaire rotatif (4) puisse mettre en prise ledit filet externe (16) du raccord Lueur femelle (2), ladite prédécoupe (13) s'ouvre complètement, et lorsque ledit écrou annulaire (4) est complètement dévissé du raccord Lueur femelle (2), ladite prédécoupe (13) se referme complètement instantanément.
